# EUROPEAN PATENT SPECIFICATION

(11) **EP 1 870 711 B1**
(45) Date of publication and mention of the grant of the patent: **24.03.2010**
(21) Application number: 06712425.5
(22) Date of filing: 26.01.2006
(51) Int. Cl.: G01N 33/68, C07K 14/82, G01N 27/62, G01N 30/00, G01N 37/00

(54) **MARKER PROTEIN FOR USE IN DIAGNOSIS OF PANCREATIC CANCER**
MARKERPROTEIN ZUR VERWENDUNG BEI DER DIAGNOSE VON BAUCHSPEICHELDRÜSENKREBS
PROTÉINE MARQUEUR UTILISÉE LORS DU DIAGNOSTIC DU CANCER DU PANCRÉAS

(30) Priority: 14.03.2005 JP 2005070512
(43) Date of publication of application: 26.12.2007
(73) Proprietor: Japan Health Sciences Foundation, Tokyo 103-0001 (JP)
(72) Inventor: HIROHASHI, Setsuo, Tokyo, 1040045 (JP); HONDA, Kazufumi, Tokyo, 1040045 (JP); YAMADA, Tesshi, Tokyo, 1040045 (JP)
(74) Representative: Ahner, Francis
(86) International application number: PCT/JP2006/301243
(87) International publication number: WO 2006/098087

(56) References cited:
- EP-A- 1 439 188
- JP-A- 2003 528 296
- JP-A- 2003 532 055
- VON EGGELING F ET AL: "MASS SPECTROMETRY MEETS CHIP TECHNOLOGY: A NEW PROTEOMIC TOOL IN CANCER RESEARCH?" ELECTROPHORESIS, DE, vol. 22, no. 14, 1 August 2001 (2001-08-01), pages 2898-2902, XP001069193 ISSN: 0173-0835
- KOOPMANN J ET AL: "Serum Diagnosis of Pancreatic Adenocarcinoma Using Surface-Enhanced Laser Desorption and Ionization Mass Spectrometry" CLINICAL CANCER RESEARCH 20040201 US, vol. 10, no. 3, 1 February 2004 (2004-02-01), pages 860-868, XP002482029 ISSN: 1078-0432
- ROSTY C ET AL: "IDENTIFICATION OF HEPATOCARCINOMA-INTESTINE-PANCREAS/PANCREA TITIS-ASS OCIATED PROTEIN I AS A BIOMARKER FOR PANCREATIC DUCTAL ADENOCARCINOMA BY PROTEIN BIOCHIP TECHNOLOGY" CANCER RESEARCH, AMERICAN ASSOCIATION FOR CANCER RESEARCH, BALTIMORE, MD, vol. 62, no. 2, 15 March 2002 (2002-03-15), pages 1868-1875, XP001179968 ISSN: 0008-5472
- CHAPMAN K: "The ProteinChip(R) Biomarker System from Ciphergen Biosystems: A novel proteomics platform for rapid biomarker discovery and validation" BIOCHEMICAL SOCIETY TRANSACTIONS, COLCHESTER, ESSEX, GB, vol. 30, no. 2, 1 April 2002 (2002-04-01), pages 82-87, XP002903940 ISSN: 0300-5127

## Description

### BACKGROUND OF THE INVENTION

### Field of the Invention

The present invention relates to a plurality of pancreatic carcinoma marker proteins having specific molecular weights and to a method for detecting pancreatic carcinoma using the marker proteins as indicators.

### Background Art

Pancreatic carcinoma is one of intractable cancers. No effective therapy for it, except for surgical operation, has so far been established, so that early treatment is important. The use of CA19-9, an existing serum tumor marker, is known as a means of diagnosing pancreatic carcinoma (Shuyo Maka Rinsho Manyuaru (Clinical Manual of Tumor Markers), Hisanao Okura, Igaku-Shoin, pp. 88-89 and pp.124-127). Further, in the diagnosis of pancreatic carcinoma, helical scan CT, magnetic resonance imaging (MRI), endoscopic ultrasonography (EUS), etc. are used in these days. However, since the symptoms of pancreatic carcinoma are not clear when the disease is in its early stages, there are still many such cases that at the time when the disease is detected, it has already progressed to an advanced stage and is hard to cure. There is therefore a need for development of a diagnostic technique with which pancreatic carcinoma can be detected accurately and easily when it is still in its early stages.

### SUMMARY OF THE INVENTION

The present inventors analyzed blood samples taken from patients with pancreatic carcinoma by means of mass spectrometry using the protein chip method, and have now found that the expression levels of a plurality of blood proteins having specific molecular weights in the blood samples are significantly different from those of the blood proteins in blood samples taken from healthy individuals and that it is possible to detect pancreatic carcinoma accurately and easily by using these blood proteins as indicators. The present invention is based on this finding.
Accordingly, an object of the present invention is to provide a method for detecting pancreatic carcinoma.

A method for detecting pancreatic carcinoma according to the present invention comprises:
determining the expression level of a blood protein having a molecular weight of 17,272±9 or 17,253±9 D in a blood sample taken from a subject, and
judging that pancreatic cancer is detected in the subject when the expression level of the blood protein in the blood sample taken from the subject is lower than that of the protein in a blood sample taken from a healthy individual.

According to the present invention, by using the blood proteins having specific molecular weights as pancreatic carcinoma markers, pancreatic carcinoma can be detected accurately and easily, which makes it possible to detect and treat the disease in its early stages. It is therefore very significant that the present invention using the blood proteins as pancreatic carcinoma markers is provided.

### BRIEF DESCRIPTION OF THE DRAWINGS

Fig. 1 shows mass spectrometric analysis charts showing the peak of the blood protein having a molecular weight of 17,272±9 (m/z) taken from a healthy subject and from a pancreatic carcinoma patient, captured by cation-exchange protein chips (C10, pH 4).
Fig. 2 shows mass spectrometric analysis charts showing the peak of the blood protein having a molecular weight of 17,253±9 (m/z) taken from a healthy subject and from a pancreatic carcinoma patient, captured by cation-exchange protein chips (C10, pH 4).
Fig. 3 shows mass spectrometric analysis charts showing the peak of the blood protein having a molecular weight of 17,253±9 (m/z) taken from a healthy subject and from a pancreatic carcinoma patient, captured by reverse-phase protein chips (H50).
Fig. 4 shows the results of U tests comparing a healthy subject group and a pancreatic carcinoma patient group, made on the ionic intensity of the mass peak of the protein having a molecular weight of 17,272±9 (m/z) captured by cation-exchange protein chips (C10, pH 4) in Examples 1 and 2.
Fig. 5 shows the results of U tests comparing a healthy subject group and a pancreatic carcinoma patient group, made on the ionic intensity of the mass peak of the protein having a molecular weight of 17,253±9 (m/z) captured by cation-exchange protein chips (C10, pH 4) in Examples 1 and 2.
Fig. 6 shows the results of U tests comparing a healthy subject group and a pancreatic carcinoma patient group, made on the ionic intensity of the mass peak of the protein having a molecular weight of 17,253±9 (m/z) captured by reverse-phase protein chips (H 50) in Examples 1 and 2.
Fig. 7 shows mass spectrometric analysis charts obtained in Example 4, showing the peak of the blood protein having a molecular weight of 17,272±9 (m/z) taken from a healthy subject and from a pancreatic carcinoma patient, captured by cation-exchange protein chips (C10, pH 4).
Fig. 8 shows mass spectrometric analysis charts obtained in Example 4, showing the peak of the blood protein having a molecular weight of 8,766±5 (m/z) taken from a healthy subject and from a pancreatic carcinoma patient, captured by cation-exchange protein chips (C10, pH 4).
Fig. 9 shows mass spectrometric analysis charts obtained in Example 4, showing the peak of the blood protein having a molecular weight of 28,080±15 (m/z) taken from a healthy subject and from a pancreatic carcinoma patient, captured by cation-exchange protein chips (C10, pH 4).
Fig. 10 shows mass spectrometric analysis charts obtained in Example 4, showing the peak of the blood protein having a molecular weight of 14,799±8 (m/z) taken from a healthy subject and from a pancreatic carcinoma patient, captured by reverse-phase protein chips (H50).
Fig. 11 is a diagram showing ROC curves and their AUC values obtained in Example 4.

### DETAILED DESCRIPTION OF THE INVENTION

Pancreatic carcinoma marker proteins according to the present invention are blood proteins whose molecular weights are 17,272±9and 17,253±9 D. Here, the molecular weight of each blood protein is the center value of the mass distribution of the blood protein in mass spectrometric analysis. The blood proteins having molecular weights of 17,272±9 and 17,253±9
D are **characterized in that** their expression levels are lower in pancreatic carcinoma patients than in healthy individuals.

The pancreatic carcinoma marker proteins of the present invention can be used as an indicator of pancreatic carcinoma either singly or in combination. Although these marker proteins make it possible to detect pancreatic carcinoma accurately even when they are used singly, the disease can be detected more accurately when two or more of them are used in combination as a multi-marker. Furthermore, the pancreatic carcinoma marker proteins according to the present invention may also be used in combination with a known pancreatic carcinoma marker, such as CA 19-9, for the detection of pancreatic carcinoma, and the present invention also encompasses such an embodiment.

Although any technique can be used to determine the expression levels of the pancreatic carcinoma marker proteins as long as it can quantify the pancreatic carcinoma marker proteins, a preferred technique is mass spectrometric analysis, and a more preferred technique is time-of-flight mass spectrometric analysis. In such mass spectrometric analysis, the ionic intensities of the mass peaks of the pancreatic carcinoma marker proteins, for example, can be used to quantify the marker proteins.

Any instrument or system can be used without limitation for the above-described mass spectrometric analysis as long as it can measure the expression levels of the pancreatic carcinoma marker proteins. Examples of instruments and systems useful herein include such instruments as a quadrupole mass spectrometer, a time-of-flight mass spectrometer, a quadrupole ion trap mass spectrometer and a Fourier transform ion cyclotron resonance mass spectrometer, and a protein chip system comprising protein chips, a time-of-flight mass spectrometer, and a computer on which software for determination and analysis is installed. Of these, a quadrupole mass spectrometer, a time-of-flight mass spectrometer, and a protein chip system are preferred.

Any energy-absorbing molecule may be used in the mass spectrometric analysis in the present invention as long as it can be used in the mass spectrometric analysis of the marker proteins according to the present invention. Examples of such molecules include sinapinic acid and α-CHCA (α-cyano-4-hydroxy cinnamic acid).

It is preferred that the above-described mass spectrometric analysis be performed by the protein chip method. The protein chip method herein refers to a technique in which a sample to be analyzed is added to protein chips, and the molecular weights of proteins contained in the sample, captured by the protein chips, are measured by means of mass spectrometry. The protein chip method makes it possible to conduct purification, identification, or the like of a plurality of proteins rapidly in a short time with high efficiency by the use of a small amount of a sample, so that it can be advantageously used in the detection of pancreatic carcinoma.

Although any type of protein chips can be used in the present invention as long as it can capture the pancreatic carcinoma marker proteins, preferred types of protein chips are reverse-phase protein chips, metal-ion-fixed protein chips, and cation-exchange protein chips. Therefore, according to one embodiment of the present invention, the pancreatic carcinoma marker proteins are captured by at least one protein chip selected from the group consisting of reverse-phase protein chips, metal-ion-fixed protein chips, and cation-exchange protein chips.
According to a more preferred embodiment of the present invention, the protein chip is a reverse-phase protein chip and/or a cation-exchange protein chip.
According to another preferred embodiment of the present invention, the protein chip is a metal-ion-fixed protein chip and/or a cation-exchange protein chip.
Further, the pancreatic carcinoma marker proteins according to the present invention are well captured by cation-exchange protein chips at a pH of 4 or 7. Therefore, according to a more preferred embodiment of the present invention, the cation-exchange protein chips are prepared to have a pH of 4 or 7.

A cation-exchange group in cation-exchange protein chips is preferably carboxyl group or a carboxyalkyl group, more preferably carboxymethyl group. Preferred specific examples of the cation-exchange protein chips include WCX 2 and CM 10 (both manufactured by Ciphergen Biosystems Inc., 14F, East Tower, Yokohama Business Park, 134, Godo-cho, Hodogaya-ku, Yokohama-shi, Kanagawa-ken, Japan), and CM 10 is more preferred. A functional group in reverse-phase protein chips is preferably at least one group selected from alkyl groups, aralkyl groups and aryl groups, more preferably at least one group having 6 to 12 carbon atoms, selected from alkyl groups, aralkyl groups and aryl groups, and most preferably an alkyl group having 6 to 12 carbon atoms. Although the above functional group may be substituted, it is preferably unsubstituted. Preferred specific examples of the reverse-phase protein chips include H4 and H50 (both manufactured by Ciphergen Biosystems Inc., Japan), and H50 is more preferred. A metal ion to be fixed to the metal-ion-fixed protein chips is preferably copper or zinc ion, more preferably copper ion. Preferred specific examples of the metal-ion-fixed protein chips include IMAC 30 (manufactured by Ciphergen Biosystems Inc., Japan).

In the protein chip method, after adding a sample to protein chips, the protein chips are, if necessary, treated with a solution, such as a buffer solution, in order to make the protein chips quickly capture proteins contained in the sample and also to remove impurities, and are then washed. The solution for use in this treatment is specifically a sodium acetate buffer solution, a tris(hydroxymethyl)aminomethane/hydrogen chloride buffer solution, a phosphoric acid buffer solution, or the like. In the case where cation-exchange protein chips are used, a buffer solution at a pH of 4 or 7 is preferably used to treat the protein chips because the pancreatic carcinoma marker proteins according to the present invention have the characteristics of being captured by cation-exchange protein chips at a pH of 4 or 7. Therefore, according to a preferred embodiment of the present invention, the cation-exchange protein chips are prepared to have a pH of 4 or 7.
When reverse-phase protein chips are used, an acetonitrile or trifluoroacetate solution is preferably used to treat the protein chips.
When metal-ion-fixed protein chips are used, a sodium phosphate, chloride, or acetate solution is preferably used to treat the protein chips.

Another aspect of the present invention is the use of a blood protein having a molecular weight of 17,272±9 or 17,253±9 D as a pancreatic carcinoma marker protein

The pancreatic carcinoma marker proteins according to the present invention are useful in accurately and easily detecting pancreatic carcinoma, as mentioned above. Therefore, a further aspect of the present invention is a method for detecting pancreatic carcinoma, comprising:
determining the expression level of a blood protein having a molecular weight of 17,272±9 or 17,253±9 D in a blood sample taken from a subject, and
judging that pancreatic cancer is detected in the subject when the expression level of the blood protein in the blood specimen taken from the subject is lower than that of the protein in a blood sample taken from a healthy individual.

In the above-described method according to the present invention, whether pancreatic carcinoma is detected or not in a subject is judged by comparing the expression levels of the pancreatic carcinoma marker proteins in a blood sample taken from the subject with those of the marker proteins in a blood sample taken from a healthy individual, as mentioned above. The expression levels of the marker proteins in a healthy individual are set beforehand as thresholds depending on race, sex, age, etc. The expression levels of the marker proteins in a healthy individual can be set by a known statistical technique with reference to the amounts of the pancreatic carcinoma marker proteins in a blood sample taken from a healthy individual, quantified by the above-described method. Specific examples of techniques of setting the expression level of each marker protein include (the mean value of pancreatic carcinoma marker protein levels in a blood sample taken from a healthy individual ± standard deviation) and ROC (Receiver Operating Characteristics) analysis, and ROC analysis is preferred. Whether pancreatic carcinoma is detected or not in a subject may also be judged by comparing the expression levels of the pancreatic carcinoma marker proteins in a blood sample taken from the subject with the thresholds established beforehand by the above statistical technique from the quantified amounts of the marker proteins in a blood sample taken from a pancreatic carcinoma patient. The present invention also encompasses this embodiment.

Further, the blood sample in the present invention is usually human-derived one. Furthermore, the blood sample in the present invention is preferably blood plasma. Any method can be used to collect blood and prepare a blood sample as long as it does not affect the measurement of the marker proteins according to the present invention, and any method known to those skilled in the art can be used.

Furthermore, the pancreatic carcinoma marker proteins according to the present invention can also be used in a method for monitoring pancreatic carcinoma in a patient. The monitoring method can be effectively used to grasp accurately the progress of pancreatic carcinoma in a patient, thereby administering a more adequate medicinal agent to the patient at more adequate timing, or subjecting the patient to adequate treatment.

Accordingly, a further aspect of the present invention is a method for monitoring pancreatic carcinoma in a patient, comprising:
determining the expression level of a blood protein having a molecular weight of 17,272±9 or 17,253±9 D in a blood sample taken from a patient, and
judging that pancreatic carcinoma has progressed when the expression level of the protein has decreased with time.

### EXAMPLES

The following example will specifically explain the present invention. However, they are not to be construed to limit the scope of the invention.

### Example 1

### Selection of Peaks

### Blood Collection

Blood was collected from 104 pancreatic carcinoma patients at National Cancer Center Hospital, Tokyo, Japan, not yet subjected to any treatment, and from 116 healthy individuals by the use of EDTA blood-collection tubes during the period between September 2002 and October 2003, and the blood plasma obtained were used for the following experiments.

### Analysis by Means of Protein Chip Method

A test sample was prepared by treating each blood plasma with 9M urea and 2% CHAPS (3-[(3-cholamidopropyl)dimethylammonio]propane-sulfonic acid). Using a workstation robot Biomech 2000 (manufactured by Beckman Coulter Co.), the sample was added to three different types of protein chips. The protein chips used are cation-exchange protein chips CM10 (manufactured by Ciphergen Biosystems, Inc., Japan), metal-ion-fixed protein chips IMAC 30 (manufactured by Ciphergen Biosystems, Inc., Japan), and reverse-phase protein chips H50 (manufactured by Ciphergen Biosystems, Inc., Japan). CM10 was washed under strict conditions using a pH 7 buffer solution for washing, or under moderate conditions using a pH 4 buffer solution for washing, in accordance with the description in the handling manual prepared by Ciphergen Biosystems, Inc. and was used in the following mass spectrometric analysis.

### Mass Spectrometric Analysis

Sinapinic acid, a laser absorbent matrix, was added to the respective chips, and the chips were then dried. Subsequently, the chips were placed in a quadrupole hybrid mass spectrometer Q-star XL (manufactured by Applied Biosystems Co.) equipped with a time-of-flight mass spectrometer, and the mass and ionization quantity of low-molecular-weight proteins and peptides of below about 2 - 40 KD were measured. The mass/ionization information from the mass spectrometer Q-star XL was converted to peak information by smoothing the mass/ionization information data with a Gaussian window function, using software for analysis Analyst QS (Applied Biosystems Co.), and then adjusting the base line and the noise level in accordance with the description in "Improving protein identification from peptide mass fingerprinting through a parameterized multi-level scoring algorithm and an optimized peak detection (Electrophoresis, 1999 Dec; 20(18): 3535-50). The data were analyzed on the conditions that the limit of inaccuracy in mass of the mass spectrometer is approximately 0.05%.

### Machine Learning

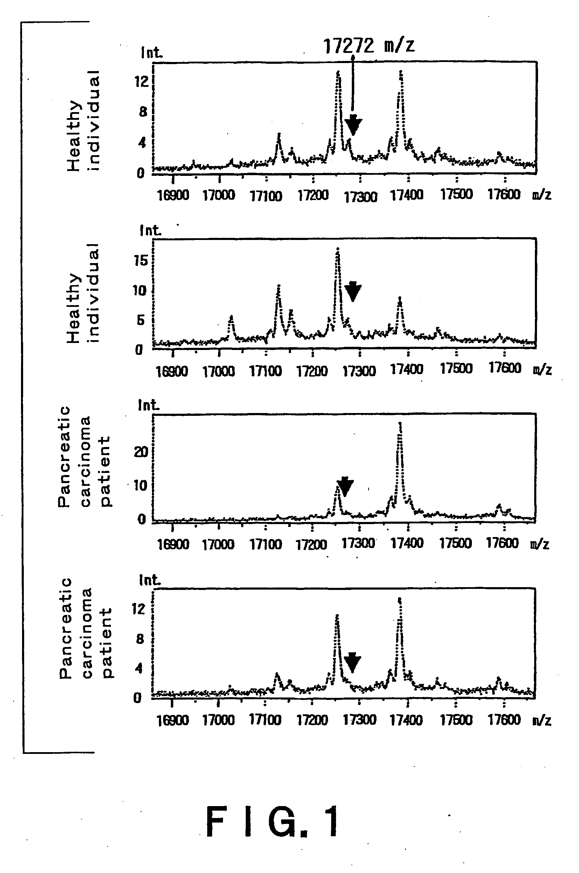

SVM (support vector machine: rbf (radius basis function) and linear), an NN (neural network), and an FNN (fuzzy neural network) were subjected to algorithmic machine learning of the peak information in the range of 2,000 to 35,000 Da obtained in the above-described manner, using as a learning set 71 pancreatic carcinoma subjects and 71 healthy individuals. The results were as shown in Fig. 1. The rates of discrimination of the three peaks at 17,272±9 (m/z) and 17,253±9 (m/z) when CM10 (pH 4) was used and at 17,253±9 (m/z) when H50 was used were above ca. 80% in every algorithmic analysis.

[Table 1]

**Table 1**

| | m/z | T test | U test | SVM | SVM | FNN | NN |
|---|---|---|---|---|---|---|---|
| | | p value | p value | (rbf) | (linear) | | |
| C10 | 17272 | 0.000 | 0.000 | 84.5 | 83.1 | 84.5 | 84.5 |
| (pH4) | | | | | | | |
| C10 | 17253 | 0.000 | 0.000 | 82.4 | 81.0 | 82.4 | 81.7 |
| (pH4) | | | | | | | |
| H50 | 17253 | 0.000 | 0.000 | 82.4 | 81.0 | 79.6 | 81.7 |

### Peak Charts

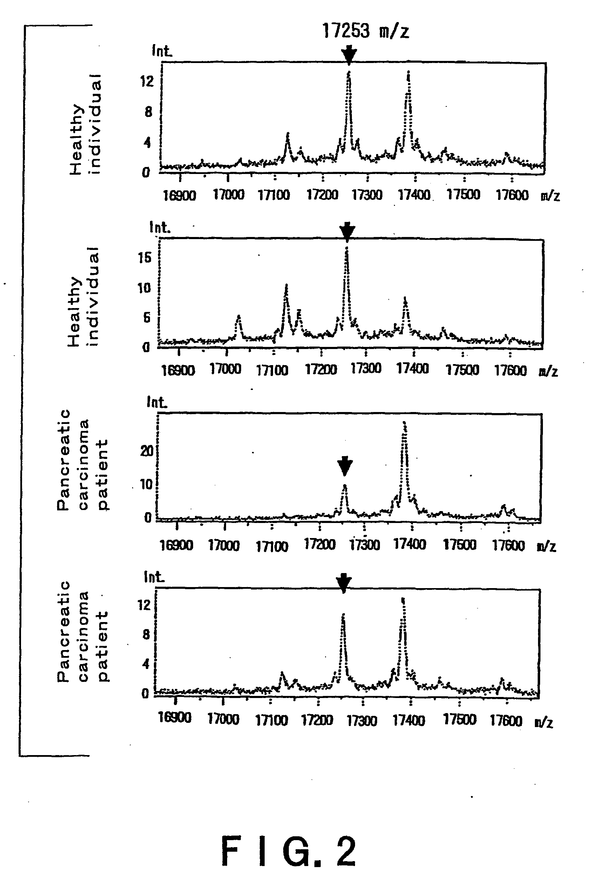

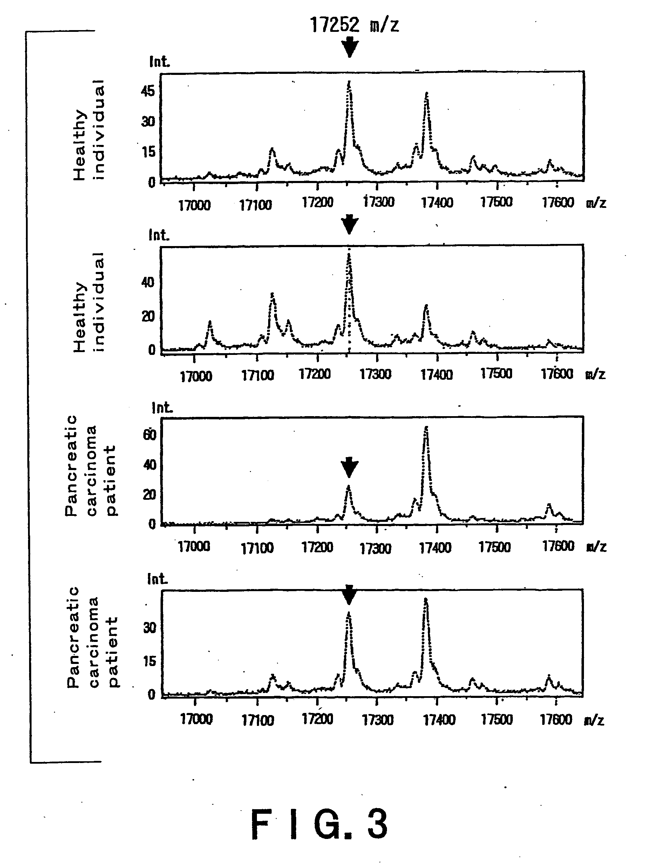

Peak charts showing the peak at 17,272±9 (m/z) when CM10 (pH4) was used, obtained from the analysis of the blood samples taken from healthy individuals and from pancreatic carcinoma patients, were as shown in Fig. 1.
Peak charts showing the peak at 17,253±9 (m/z) when CM10 (pH4) was used were as shown in Fig. 2.
Peak charts showing the peak at 17,253±9 (m/z) when H50 was used were as shown in Fig. 3.
The peak charts shown in Figs. 1 to 3 plot relative ionic intensity as the ordinate.
All the three peaks were lower in the pancreatic carcinoma patients than in the healthy individuals.

### Example 2

### Verification Tests

In terms of the three peaks found in Example 1, mass spectrometric analysis was made with the same technique as in Example 1, using, as a verification set, 33 pancreatic carcinoma patients and 45 healthy individuals who were different from those in Example 1. The rate of right answer for pancreatic carcinoma was analyzed in terms of the peaks at 17,272±9 and 17,253±9 (m/z) when CM10 (pH4) was used and the peak at 17,253±9 (m/z) when H50 was used.
As for the peak at 17,272±9 (m/z) when CM10 (pH4) was used, the rate of discrimination was 72.2%, the sensitivity, 58.8%, and the specificity, 82.2%.
As for the peak at 17,253±9 (m/z) when CM10 (pH4) was used, the rate of discrimination was 79.7%, the sensitivity, 85.3%, and the specificity, 75.6%.
As for the peak at 17,253±9 (m/z) when H50 was used, the rate of discrimination was 79.7%, the sensitivity, 82.4%, and the specificity, 77.8%.

### Verification by Means of U Tests (Mann-Whitney U Tests)

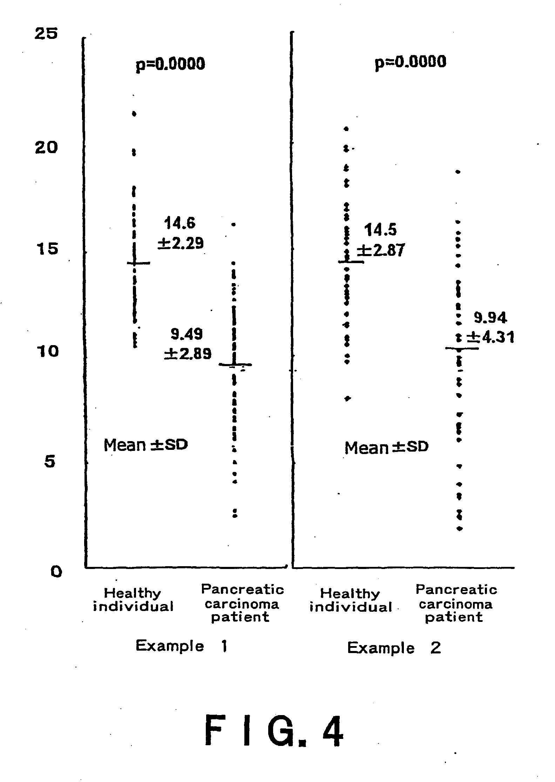

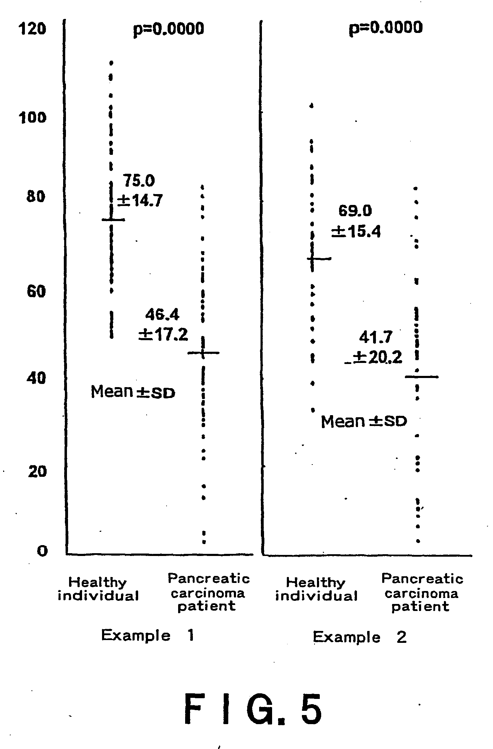

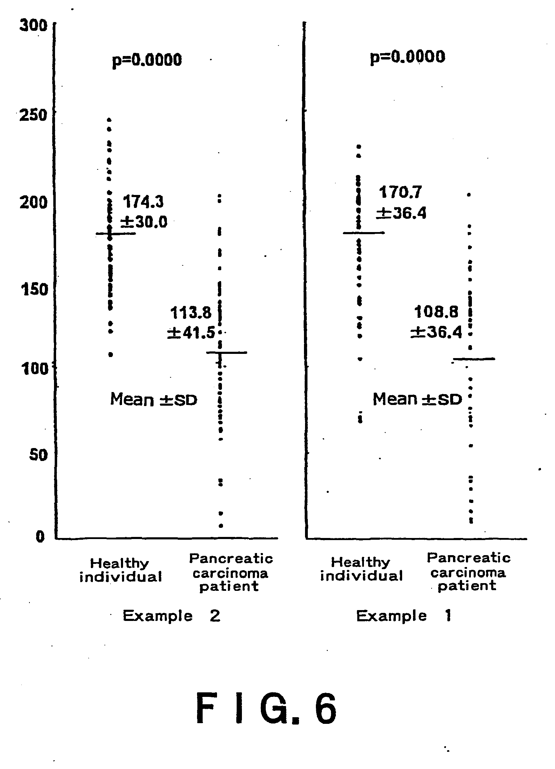

U tests comparing the healthy individual group and the pancreatic carcinoma patient group in Example 1 or 2 were made on the ionic intensities of the three mass peaks found in Example 1.
The results concerning the peak at 17,272±9 (m/z) when CM10 (pH4) was used were as shown in Fig. 4.
The results concerning the peak at 17,253±9 (m/z) when CM10 (pH4) was used were as shown in Fig. 5.
The results concerning the peak at 17,253±9 (m/z) when H50 was used were as shown in Fig. 6.
The charts shown in Figs. 4 to 6 plot relative ionic intensity as the ordinate.
The ionic intensities of all the three peaks were significantly lower in the pancreatic carcinoma patient group than in the healthy subject group.
Moreover, the results obtained from the two groups in Example 1 and those obtained from the two groups in Example 2 showed the same tendency.

### Example 3

In terms of the ionic intensity of the mass peak at 28,080±15, 17,272±9, 17,253±9, 8,766±5, or 14,779±8 (m/z) detected by the mass spectrometric analysis in Example 1, statistical analysis was made by means of T test. The results were as shown in Table 2. [Table 2]

**Table 2**

| | | Example1 (n=220) | | |
|---|---|---|---|---|
| | | Peak ionic intensity (mean value) | | p value |
| Peak(m/z) | Protein chips | Pancreatic carcinoma patients (n=104) | Healthy individuals (n=116) | |
| 28,080 | CM10(PH4) | 107 | 123 | 0.000 |
| | IMAC 30 | 23.5 | 30.3 | 0.000 |
| 17,272 | CM10(PH4) | 9.6 | 14.6 | 0.000 |
| | H50 | 42 | 64 | 0.000 |
| 17,252 | CM10(PH4) | 45 | 73 | 0.000 |
| | H50 | 113 | 175 | 0.000 |
| 14,779 | CM10(PH7) | 13.9 | 10.4 | 0.000 |
| | H50 | 11.3 | 7.1 | 0.000 |
| 8,766 | CM10(PH4) | 7.3 | 12.6 | 0.000 |
| | H50 | 13.1 | 18.8 | 0.000 |
| | IMAC 30 | 8.0 | 15.3 | 0.000 |

In the case where the cation-exchange protein chips (CM10, pH 4) were used, the ionic intensities of the mass peaks at molecular weights of 28,080±15, 17,272±9, 17,253±9, and 8,766±5 (m/z) were significantly lower in the pancreatic cancer patient group than in the healthy individual group, while the ionic intensity of the mass peak at 14,779±8 (m/z) was significantly higher in the pancreatic cancer patient group than in the healthy individual group. Further, when the reverse-phase protein chips (H50) were used, the peak intensities of the mass peaks at 17,272±9, 17,253±9, and 8,766±5 (m/z) were significantly lower in the pancreatic cancer patient group than in the healthy individual group, and the peak intensity of the mass peak at 14,779±8 (m/z) was significantly higher in the pancreatic cancer patient group than in the healthy individual group. Furthermore, when the metal-ion-fixed protein chips (IMAC30, protein chips of copper-ion-fixed type) were used, the peak intensities of the mass peaks at 28,080±15 and 8,766±5 (m/z) were significantly lower in the pancreatic cancer patient group than in the healthy individual group.

### Example 4

### Mass Spectrometric Analysis

Samples were prepared from blood collected from 71 pancreatic carcinoma patients at National Cancer Center Hospital, Tokyo, Japan, not yet subjected to any treatment, and from 71 healthy individuals and the samples were mass analyzed with the same technique as in Example 1. Cation-exchange protein chips CM10 (manufactured by Ciphergen Biosystems, Inc., Japan) and reverse-phase protein chips H50 (manufactured by Ciphergen Biosystems, Inc., Japan), were used in the analysis. CM10 was washed under moderate conditions using a pH 4 buffer solution for washing, in accordance with the description in the handling manual prepared by Ciphergen Biosystems, Inc.

### Machine Learning

An rbf SVM was subjected to algorithmic machine learning, using the peak information obtained from the above-described mass spectrometric analysis as the learning set. As a result, peaks were detected at 28,080±15, 17,272±9 and 8,766±5 (m/z) when CM10 (pH 4) was used and at 14,779±8 (m/z) when H50 was used.

### Peak Charts

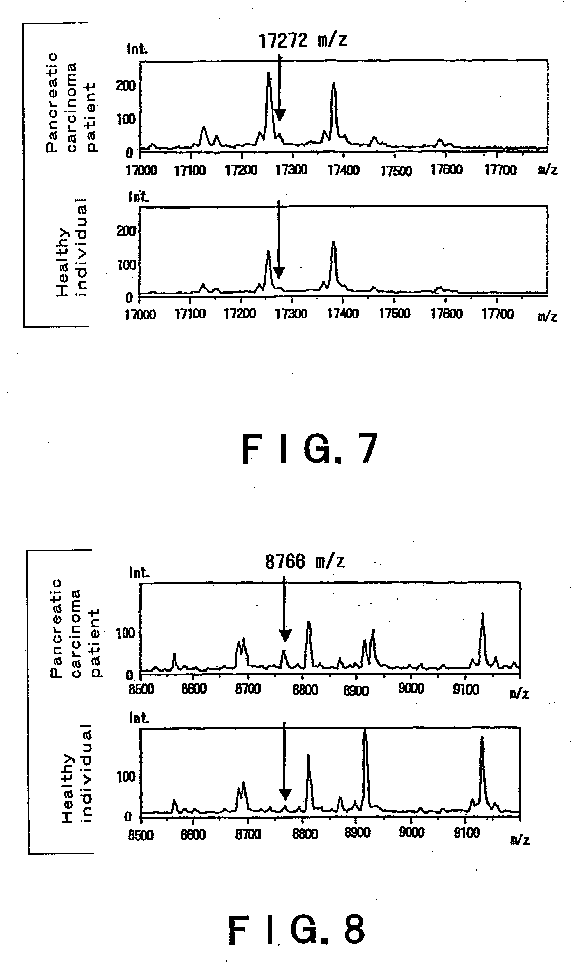

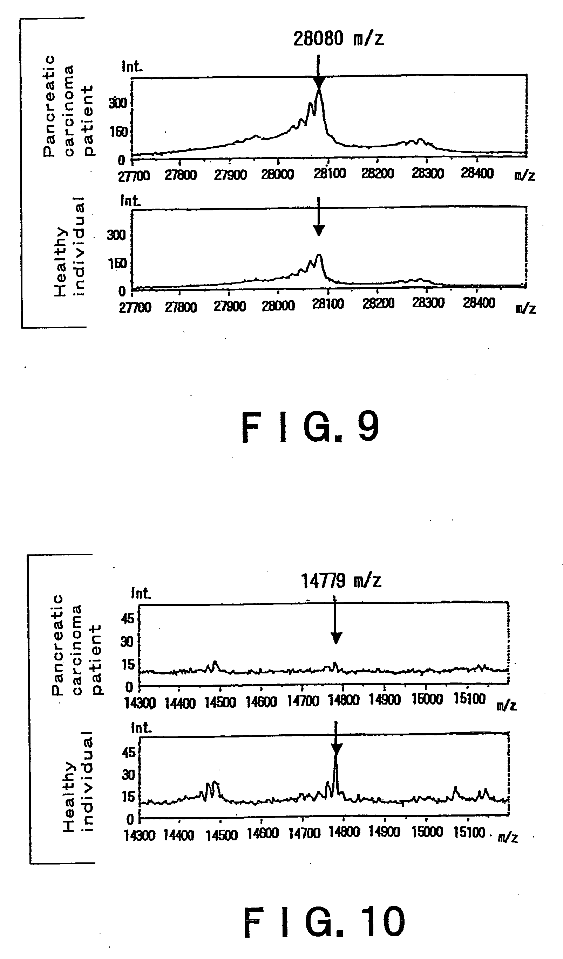

Peak charts obtained from the analyses in Example 4 were as shown in Figs. 7 to 10. The peak charts plot relative ionic intensity as the ordinate.
As shown in Figs. 7 to 9, the intensities of the peaks at 17,272±9, 8,766±5, and 28,080±15 (m/z) when CM10 (pH 4) was used were lower in the pancreatic carcinoma patient group than in the healthy individual group. On the other hand, as shown in Fig. 10, the intensity of the peak at 14,779±8 (m/z) when H50 was used was higher in the pancreatic carcinoma patient group than in the healthy subject group.

### ROC Curves

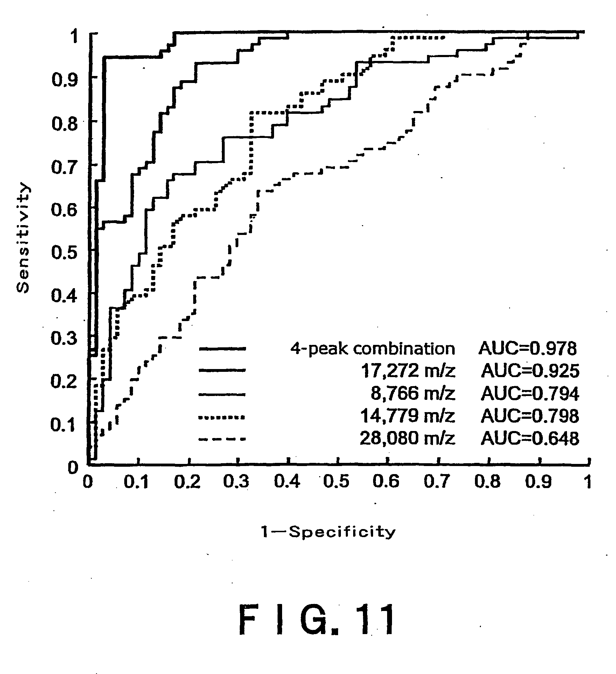

The ROC curves of the above four peaks, and their AUC values were as shown in Fig. 11. When all the above four peaks were detected (4-peak combination), the sensitivity was 97.2%, the specificity, 94.4%, and the AUC value, 0.978. With respect to the four peaks, the discrimination power was confirmed also by means of LOO (leave-one-out) cross validation.

### Example 5

### Verification Tests

Verification tests were carried out with the same technique as in Example 4, using as a verification set 33 pancreatic carcinoma patients, not yet subjected to any treatment, and 45 healthy subjects, who were different from those in Example 4. As a result, when all the four peaks found in Example 4 were detected, the rate of discrimination was 91.0%, the sensitivity, 90.9%, and the specificity, 91.1%.

### Verification by Means of U Tests

U tests comparing the healthy individual group with the pancreatic carcinoma patient group in Example 4 or 5 were made on the peak intensities of the above-described four mass peaks.
The results of the U tests were as shown in Table 3. Each statistically obtained value shown in the table is (the mean ionic intensity of the peak) ± S.D.

[Table 3]

**Table 3**

| | | Example 4 (n=142) | | | Example 5 (n=78) | | |
|---|---|---|---|---|---|---|---|
| Protein chips | Peak (m/z) | Pancreatic carcinoma patients | Healthy individuals | p value | Pancreatic carcinoma patients | Healthy individuals | p value |
| | | (n=71) | (n=71) | | (n=33) | (n=45) | |
| CM10 (PH4) | 17,272 | 9.49±2.88 | 14.6±2.29 | 0.0000 | 9.74±4.22 | 14.5±2.29 | 0.0000 |
| | 8,766 | 7.65±3.53 | 12.1±5.55 | 0.0000 | 7.04±4.39 | 13.4±5.81 | 0.0000 |
| | 28,080 | 113±36.7 | 132±33.5 | 0.0022 | 92.4±24.3 | 110±21.6 | 0.0078 |
| H50 | 14,779 | 11.8±4.43 | 7.85±3.68 | 10.4±3.85 | 0.0000 | 6.46±1.63 | 0.00000 |

The results of the U tests of the peak intensities in Example 4 and those of the U tests of the peak intensities in Example 5 showed the same tendency.
The peak intensities of all the peaks at 17,272±9, 8,766±5, and 28,080±15 (m/z) detected when CM10 (pH 4) was used were significantly lower in the pancreatic carcinoma patient group than in the healthy individual group. On the other hand, the peak intensity of the peak at 14,779±8 (m/z) detected when H50 was used was significantly higher in the pancreatic carcinoma patient group than in the healthy individual group.

### Example 6

### Detection of Pancreatic Carcinoma Using CA19-9 and Mass Peaks as Indicator

With the same technique as in Example 1, samples were prepared at Tokyo Medical University Hospital, Japan from blood collected from 29 pancreatic carcinoma patients, not yet subjected to any treatment, and from 39 healthy individuals and the samples were mass analyzed. Peaks were detected at 17,272±9, 8,766±5, and 28,080±15 (m/z) when CM10 (pH 4) was used, and at 14,779±8 (m/z) when H50 was used. Further, the detection of CA19-9 in the blood plasma taken from the pancreatic carcinoma patients and the healthy individuals was made using a CA19-9 radioimmunoassay kit (manufactured by FUJIREBIO INC., Japan). Setting the cut-off value of blood plasma CA19-9 to 37 U/ml, the detection of pancreatic carcinoma was made.

The results were as shown in Table 4. In Table 4, SELDI refers to the case where all the peaks at 17,272±9, 8,766±5, and 28,080±15 (m/z) when CM10 (pH 4) was used and at 14,779±8 (m/z) when H50 was used were detected. "Combination" in the table refers to the case where both SELDI and CA19-9 were detected. "n" is a number of the subjects in which CA 19-9, SELDI, or Combination was found. "Clinical stage" is the stages of the disease classified according to the description in the handling rules for pancreatic carcinoma, edited by the Japan Pancreatic Society.
[Table 4]

**Table 4**

| | number of subjects | CA19-9, n (%) | SELDI, n (%) | Combination, n (%) |
|---|---|---|---|---|
| Healthy subjects | 39 | 3(7.7) | 4(10.3) | 6(15.4) |
| Pancreatic carcinoma patients | 29 | 25(86.2) | 26(89.7) | 29(100) |
| Clinical stage | | | | |
| I | 1 | 1(100) | 1(100) | 1(100) |
| II | 3 | 3(100) | 2(66.6) | 3(100) |
| III | 1 | 0(0) | 1(100) | 1(100) |
| IV | 24 | 21(87.5) | 22(91.7) | 24(100) |

Pancreatic carcinoma was detected in 100% of the pancreatic carcinoma patients when the peaks at 17,272±9, 8,766±5, and 28,080±15 (m/z) when CM10 (pH 4) was used, the peak at 14,779±8 (m/z) when H50 was used, and CA19-9 were employed together as indicators.

## Claims

1. A method for detecting pancreatic carcinoma, comprising:
determining the expression level of a blood protein having a molecular weight of 17,272±9 or 17,253±9(D) in a blood sample taken from a subject, and
judging that pancreatic cancer is detected in the subject when the expression level of the blood protein in the blood sample taken from the subject is lower than that of the protein in a blood sample taken from a healthy individual.

2. A method for monitoring pancreatic carcinoma in a patient, comprising:
determining the expression level of a blood protein having a molecular weight of 17,272±9 or 17,253±9 (D) in a blood sample taken from the patient, and
judging that pancreatic cancer has progressed when the expression level of the protein has decreased with time.

3. The method according to claim 1 or 2, wherein the expression level of the blood protein is lower in pancreatic carcinoma patients than in healthy individuals.

4. The method according to claim 3, wherein the determination of the expression level of the blood protein is made by mass spectrometric analysis.

5. The method according to claim 4, wherein the mass spectrometric analysis is time-of-flight mass spectrometric analysis.

6. The method according to claim 4 or 5, wherein the mass spectrometric analysis is performed by a protein chip method.

7. The method according to claim 6, wherein the blood protein is captured by at least one protein chip selected from the group consisting of reverse-phase protein chips, metal-ion-fixed protein chips, and cation-exchange protein chips.

8. The method according to claim 7, wherein the protein chip is a reverse-phase protein chip, and/or a cation-exchange protein chip.

9. The method according to claim 8, wherein the cation-exchange protein chip is prepared to have a pH of 4 or 7.

10. Use of a blood protein having a molecular weight of 17,272±9 or 17,253±9 (D) as pancreatic carcinoma marker.

11. The use according to claim 10, wherein the expression level of the blood protein is lower in pancreatic carcinoma patients than in healthy individuals.

12. The use according to claim 11, wherein the determination of the expression level of the blood protein is made by mass spectrometric analysis.

13. The use according to claim 12, wherein the mass spectrometric analysis is time-of-flight mass spectrometric analysis.

14. The use according to claim 12 or 13, wherein the mass spectrometric analysis is performed by a protein chip method.

15. The use according to claim 14, wherein the blood protein is captured by at least one protein chip selected from the group consisting of reverse-phase protein chips, metal-ion-fixed protein chips, and cation-exchange protein chips.

16. The use according to claim 15, wherein the protein chip is a reverse-phase protein chip, and/or a cation-exchange protein chip.

17. The use according to claim 16, wherein the cation-exchange protein chip is prepared to have a pH of 4 or 7.

## Patentansprüche

1. Verfahren zum Nachweisen von Bauchspeicheldrüsenkrebs, umfassend:
Bestimmen des Expressionsspiegels eines Blutproteins mit einer relativen Molekülmasse von 17.272±9 oder 17.253±9 (D) in einer Blutprobe, die einem Probanden entnommen wurde, und
Urteilen, dass Bauchspeicheldrüsenkrebs beim Probanden nachgewiesen ist, wenn der Expressionsspiegel des Blutproteins in der dem Probanden entnommenen Blutprobe niedriger als der des Proteins in einer Blutprobe ist, die einer gesunden Person entnommen wurde.

2. Verfahren zum Überwachen von Bauchspeicheldrüsenkrebs bei einem Patienten, umfassend:
Bestimmen des Expressionsspiegels eines Blutproteins mit einer relativen Molekülmasse von 17.272±9 oder 17.253±9 (D) in einer dem Patienten entnommenen Blutprobe, und
Urteilen, dass der Bauchspeicheldrüsenkrebs fortgeschritten ist, wenn der Expressionsspiegel des Proteins mit der Zeit gesunken ist.

3. Verfahren nach Anspruch 1 oder 2, wobei der Expressionsspiegel des Blutproteins bei Patienten mit Bauchspeicheldrüsenkrebs niedriger als bei gesunden Personen ist.

4. Verfahren nach Anspruch 3, wobei die Bestimmung des Expressionsspiegels des Blutproteins durch massenspektrometrische Analyse erfolgt.

5. Verfahren nach Anspruch 4, wobei es sich bei der massenspektrometrischen Analyse um eine flugzeitmassenspektrometrische Analyse handelt.

6. Verfahren nach Anspruch 4 oder 5, wobei die massenspektrometrische Analyse durch ein Proteinchip-Verfahren erfolgt.

7. Verfahren nach Anspruch 6, wobei das Blutprotein durch wenigstens einen Proteinchip eingefangen wird, der aus der Gruppe bestehend aus Umkehrphasen-Proteinchips, metallionengebundenen Proteinchips und Kationenaustausch-Proteinchips ausgewählt ist.

8. Verfahren nach Anspruch 7, wobei der Proteinchip ein Umkehrphasen-Proteinchip und/oder ein Kationenaustausch-Proteinchip ist.

9. Verfahren nach Anspruch 8, wobei der Kationenaustausch-Proteinchip so ausgelegt is, dass er einen pH von 4 oder 7 aufweist.

10. Verwendung eines Blutproteins mit einer relativen Molekülmasse von 17.272±9 oder 17.253±9 (D) als Marker für Bauchspeicheldrüsenkrebs.

11. Verwendung nach Anspruch 10, wobei der Expressionsspiegel des Blutproteins bei Patienten mit Bauchspeicheldrüsenkrebs niedriger als bei gesunden Personen ist.

12. Verwendung nach Anspruch 11, wobei die Bestimmung des Expressionsspiegels des Blutproteins durch massenspektrometrische Analyse erfolgt.

13. Verwendung nach Anspruch 12, wobei es sich bei der massenspektrometrischen Analyse um eine flugzeitmassenspektrometrische Analyse handelt.

14. Verwendung nach Anspruch 12 oder 13, wobei die massenspektrometrische Analyse durch ein Proteinchip-Verfahren erfolgt.

15. Verwendung nach Anspruch 14, wobei das Blutprotein durch wenigstens einen Proteinchip eingefangen wird, der aus der Gruppe bestehend aus Umkehrphasen-Proteinchips, metallionengebundenen Proteinchips und Kationenaustausch-Proteinchips ausgewählt ist.

16. Verwendung nach Anspruch 15, wobei der Proteinchip ein Umkehrphasen-Proteinchip und/oder ein Kationenaustausch-Proteinchip ist.

17. Verwendung nach Anspruch 16, wobei der Kationenaustausch-Proteinchip so ausgelegt is, dass er einen pH von 4 oder 7 aufweist.

## Revendications

1. Procédé de détection d'un carcinome pancréatique, comprenant les étapes consistant à :
déterminer le niveau d'expression d'une protéine du sang ayant un poids moléculaire de 17 272±9 ou de 17 253±9(D) dans un échantillon de sang prélevé sur un sujet, et
considérer que le cancer du pancréas est détecté chez le sujet quand le niveau d'expression de la protéine du sang dans l'échantillon de sang prélevé sur le sujet est inférieur à celui de la protéine dans un échantillon de sang prélevé sur un individu sain.

2. Procédé de suivi d'un carcinome pancréatique chez un patient, comprenant les étapes consistant à :
déterminer le niveau d'expression d'une protéine du sang ayant un poids moléculaire de 17 272±9 ou de 17 253±9(D) dans un échantillon de sang prélevé sur le sujet, et
considérer que le cancer du pancréas a progressé quand le niveau d'expression de la protéine du sang a baissé avec le temps.

3. Procédé selon les revendications 1 ou 2, dans lequel le niveau d'expression de la protéine du sang est plus bas chez les patients atteints d'un carcinome pancréatique que chez les individus sains.

4. Procédé selon la revendication 3, dans lequel la détermination du niveau d'expression de la protéine du sang est effectuée par une analyse par spectrométrie de masse.

5. Procédé selon la revendication 4, dans lequel l'analyse par spectrométrie de masse est une analyse par spectrométrie de masse à temps de vol.

6. Procédé selon les revendications 4 ou 5, dans lequel l'analyse par spectrométrie de masse est effectuée par un procédé sur puce à protéine.

7. Procédé selon la revendication 6, dans lequel la protéine du sang est capturée par au moins une puce à protéine choisie dans le groupe constitué par les puces à protéine en phase inverse, les puces à protéine fixée par ions métalliques, et les puces à protéine du type par échange de cations.

8. Procédé selon la revendication 7, dans lequel la puce à protéine est une puce à protéine en phase inverse et/ou une puce à protéine du type par échange de cations.

9. Procédé selon la revendication 8, dans lequel la puce à protéine du type par échange de cations est préparée pour présenter un pH de 4 ou 7.

10. Utilisation d'une protéine du sang ayant un poids moléculaire de 17 272±9 ou de 17 253±9(D) à titre de marqueur du carcinome pancréatique.

11. Utilisation selon la revendication 10, dans laquelle le niveau d'expression de la protéine du sang est plus bas chez les patients atteints d'un carcinome pancréatique que chez les individus sains.

12. Utilisation selon la revendication 11, dans laquelle la détermination du niveau d'expression de la protéine du sang est effectuée par une analyse par spectrométrie de masse.

13. Utilisation selon la revendication 12, dans laquelle l'analyse par spectrométrie de masse est une analyse par spectrométrie de masse à temps de vol.

14. Utilisation selon les revendications 12 ou 13, dans laquelle l'analyse par spectrométrie de masse est effectuée par un procédé sur puce à protéine.

15. Utilisation selon la revendication 14, dans laquelle la protéine du sang est capturée par au moins une puce à protéine choisie dans le groupe constitué par les puces à protéine en phase inverse, les puces à protéine fixée par ions métalliques, et les puces à protéine du type par échange de cations.

16. Utilisation selon la revendication 15, dans laquelle la puce à protéine est une puce à protéine en phase inverse et/ou une puce à protéine du type par échange de cations.

17. Utilisation selon la revendication 16, dans laquelle la puce à protéine du type par échange de cations est préparée pour présenter un pH de 4 ou 7.
